# EUROPÄISCHE PATENTANMELDUNG

(11) **EP 0 829 542 A1**
(43) Veröffentlichungstag der Anmeldung: **18.03.1998**
(21) Anmeldenummer: 97115521.3
(22) Anmeldetag: 08.09.1997
(51) Int. Cl.: C12Q 1/68

(54) **Verfahren zur Amplifikation von Nukleinsäuren**

(30) Priorität: 13.09.1996 DE 19637339
(71) Anmelder: HOECHST AKTIENGESELLSCHAFT, 65929 Frankfurt am Main (DE)
(72) Erfinder: Uhlmann, Eugen, Dr., 61479 Glashütten (DE); Breipohl, Gerhard, Dr., 60529 Frankfurt (DE); Benner, Steven, Prof,-Dr., Gainesville, FL 32605 (US); Lutz, Michael, DCh., 77654 Offenburg (DE)

(57) **Zusammenfassung**

Die vorliegende Erfindung betrifft ein neues vorteilhaftes Verfahren zur Amplifikation von Nukleinsäuren unter Verwendung von DNA/PNA-Primern und eines temperaturstabilen Polymeraseenzyms.

## Beschreibung

Die vorliegende Erfindung betrifft ein neues vorteilhaftes Verfahren zur Amplifikation von Nukleinsäuren unter Verwendung von DNA/PNA-Primern und eines temperaturstabilen Polymeraseenzyms.

Kürzlich wurden Polyamid-Nukleinsäure-Derivate (PNAs) beschrieben (Michael Egholm, Peter E. Nielsen, Rolf H. Berg and Ole Buchardt, Science (1991) 254, 1497-1500; WO 92/20702; M. Egholm et al. Nature (1993) 365, 566-568; P. Nielsen, Bioconjugate Chem. (1994) 5, 3-7), die mit höherer Affinität als natürliche Oligonucleotide an komplementäre Zielsequenzen (DNA oder RNA) binden. In diesen sogenannten PNAs ist das Desoxyribose-Phosphat-Gerüst durch ein Polyamid-Oligomer ersetzt.

Es wurde gefunden, daß PNAs von DNA Polymerasen nicht als Primer akzeptiert werden (H. Ørum, et al., Nucl. Acids Res. (1993) 21, 5332-5336; D. B. Demers, et al., Nucl. Acids Res. (1995) 23, 3050-5) und somit nicht als Primer zur Amplifikation von Nukleinsäuren mit Hilfe von DNA-Polymerasen eingesetzt werden können.

In EP-A 0672 677 und WO 95/08556 werden PNA/DNA Hybridmoleküle beschrieben, die unter anderem auch als Primer zur Amplifikation von Nukleinsäuren unter Verwendung von Klenow-Polymerasen eingesetzt werden können.

Ein Vorteil der Verwendung von PNA/DNA-Primern statt natürlicher Oligonucleotid-Primer zur Amplifikation von Nukleinsäuren besteht darin, daß der mit Hilfe des PNA-Primers kopierte Nucleinsäurestrang am 5'-Ende einen PNA-Teil enthält, der gegen 5'-Exonucleasen stabil ist. Somit können alle natürlichen DNA- bzw. RNA-Sequenzen im Reaktionsgemisch durch 5'-Exonucleasen abgebaut werden, ohne daß der PNA-haltige Strang angegriffen wird.

Jedoch gelingen die in WO 95/08556 beschriebenen Polymerasereaktionen mit Klenow-Polymeraseenzymen nur dann, wenn die dort beschriebenen PNA/DNA-Primer mindestens vier Nucleotide am 3'-Terminus aufweisen, während ein PNA/DNA-Primer mit nur zwei Nucleotiden am 3'-Terminus in der DNA Polymerasereaktion versagt.

Aus EP-A 0672 677 ist bekannt, daß PNA/DNA-Hybride mit nur einem 5'-Deoxy-5'-aminonukleosid am Carboxyterminus als Primer eingesetzt werden können, wenn zur enzymatischen Polymerisierung die DNA-Polymerase von E. coli (Klenow Fragment) eingesetzt wird.

Diese Ergebnisse sind völlig unerwartet, da aus Kristallstruktur-Studien an DNA Polymerase/Primer-Template-Komplexen hervorgeht, daß an mehreren Positionen Wechselwirkungen zwischen den Phosphodiester-Funktionen des Primers und dem Enzym bestehen (C. M. Joyce, T. A. Steitz, Annu. Rev. Biochem. (1994) 63, 777-822; S. H. Eom, Nature (1996) 382, 278-281).

Daß die Eliminierung der negativen Ladungen im Primer der Akzeptanz des Primers durch Polymerasen entgegenwirkt, wurde zudem durch Untersuchungen mit Primern bestätigt, in denen die natürlichen Phosphodiesterfunktionen partiell durch neutrale Internukleosidbrücken, wie beispielsweise 3'-O-Sulfonat oder 3'-N-Sulfonamid ersetzt waren (K. A. Perrin et al., J. Am. Chem. Soc. (1994) 116, 7427-7428).

Aufgrund dieser Ergebnisse war anzunehmen, daß das Klenow-Enzym hinsichtlich seiner teilweisen Akzeptanz ungeladener Primer, um die es sich ja bei den in EP-A 0672 677 beschriebenen PNA/DNA-Hybridmolekülen mit einem 5'-Deoxy-5'-aminonukleosid am Carboxyterminus handelt, eine Ausnahme unter den verschiedenen DNA-Polymerasen darstellt.

Ein Nachteil der Verwendung der Klenow-Polymerase zur Amplifikation von Nukleinsäuren unter Einsatz von PNA/DNA-Primern ist, daß dieses Enzym nicht so temperaturstabil ist, daß beispielsweise eine Amplifikation des kopierten Templatestranges analog bekannter Amplifikationstechniken wie PCR und LCR erreicht werden kann.

Aufgabe der vorliegende Erfindung war es daher, andere DNA-Polymerasen zu finden, die PNA/DNA-Primer elongieren ohne die obengenannten Nachteile aufzuweisen.

Überraschenderweise wurde nun gefunden, daß temperaturstabile DNA-Polymerasen PNA/DNA-Primer, die an einem Ende mindestens eine, gegebenenfalls modifizierte Nukleosid-Einheit mit einer 3 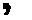 -Hydroxygruppe tragen, elongieren können.

Die vorliegende Erfindung betrifft somit ein Verfahren zur Amplifikation von Nukleinsäuren mit Hilfe eines Polymeraseenzyms und eines oder mehrerer PNA/DNA-Primer, die an einem Ende mindestens eine Nukleosid-Einheit mit einer 3'-Hydroxygruppe haben sowie den üblicherweise zur Amplifikation von Nukleinsäuren benötigten Komponenten wie z.B. unmarkierte oder radioaktiv markierte Desoxyribonukleotidtriphosphate, dadurch gekennzeichnet, daß das Polymeraseenzym temperaturstabil ist.
Geeignet sind z.B. die Tth™ DNA-Polymerase aus Thermus sp. (Thermus thermophilus), die Pwo DNA-Polymerase aus Pyrococcus woesei, die 9°N DNA-Polymerase™ aus Thermococcus sp. strain 9°N oder die Vent™ -Polymerase aus Thermococcus litoralis.

In einer weiteren Ausführungsform der Erfindung wird in dem Verfahren mehr als ein Polymerasetyp eingesetzt. Beispielsweise kann eine Kombination von zwei oder mehr unterschiedlichen Typen von Polymerasen verwendet werden. Eine spezielle Ausführungsform der Erfindung betrifft ein Verfahren, bei dem eine Kombination von Vent™ DNA-Polymerase und Tth™ DNA-Polymerase verwendet wird.

Beispiele für geeignete PNA/DNA-Primer sind in EP 0 622 677 A2 beschrieben. Insbesondere sind die in EP 0 622 677 A2 beschriebenen PNA/DNA Primer geeignet, wenn sie an einem Ende ein bis drei, bevorzugt eine Nukleosid-Einheit aufweisen.

Bevorzugt sind auch solche PNA/DNA-Primer, bei denen sich die Nukleosideinheiten am 3'-Ende des PNA/DNA-Primers befinden.

Geeignet sind beispielsweise PNA/DNA Primer der Formel I 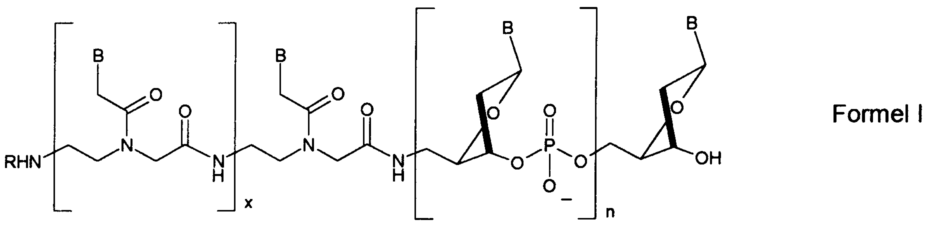 worin
- n: 0 - 2 ist,
- x: 2 - 50 ist und
- R: Wasserstoff oder ein organischer Rest mit 2-18 Kohlenstoffatomen ist, und
- B: unabhängig voneinander für eine in der Nukleotidchemie übliche Base, beispielsweise für eine natürliche Base wie Adenin, Cytosin, Thymin, Guanin, Uracil, Hypoxanthin oder unnatürliche Base wie beispielsweise Purin, 2.6-Diaminopurin, 7-Deazaguanin, 7-Deazaadenin, N⁴N⁴-Ethanocytosin, N⁶N⁶-Ethano-2.6-diaminopurin, Pseudoisocytosin, 5-Methylcytosin, 5-Fluoruracil, 5-(C₃-C₆)-Alkinyluracil, 5-(C₃-C₆)-Alkinylcytosin oder deren Prodrugformen steht.

Geeignet sind des weiteren DNA/PNA-Primer der Formel II oder III 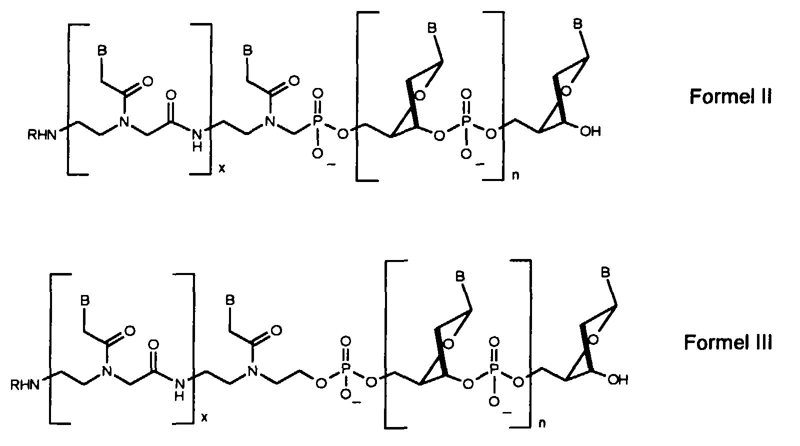 worin
- n: 0 - 2 ist,
- x: 2 - 50 ist und
- R: Wasserstoff oder ein organischer Rest mit 2-18 Kohlenstoffatomen ist, und B die oben genannte Bedeutung hat.

Bevorzugt sind DNA/PNA-Primer der Formel I, II oder III, in denen n = 0 ist. Bevorzugt sind auch DNA/PNA-Primer der Formel I, II oder III, in denen x 5-25 besonders bevorzugt 10 - 18 ist.

Beispiele für einen organischen Rest mit 2-18 Kohlenstoffatomen sind C₂-C₁₈-Alkanoyl, bevorzugt C₂-C₁₀-Alkanoyl, besonders bevorzugt Acetyl, C₂-C₁₈-Alkyl, bevorzugt C₂-C₁₀-Alkyl, C₃-C₈ -Cycloalkanoyl oder C₃-C₁₃-Heteroaryl.

Unter dem Begriff Nukleosideinheit wird ein Nukleosid verstanden, das einen Zuckerrest und eine Base aufweist, wobei die Base und der Zuckerrest modifiziert sein kann und wobei die einzelnen Nukleosideinheiten über einen Phosphatrest miteinander verknüft sind und wobei ferner die Nukleosideinheiten mit dem PNA-Teil des Primers über einen Phosphatrest oder eine Amidbindung verknüpft sind. Bei dem Zuckerrest handelts es sich üblicherweise um einen Riboserest, wobei dieser jedoch auch modifiziert sein kann.

Als Beispiele für Nukleoside, die Modifikationen am Zuckerrest aufweisen seien genannt:
5'-Amino-5'-deoxythymidin, Ethylamino-N-(thyminylacetyl)ethanol, [(2-Aminoethyl)-N9-adenylacetylamino]methylphosphonsäure.

Unter Basen sind übliche Basen, wie beispielsweise die natürlichen Basen Adenin, Cytosin, Thymin, Guanin, Uracil, Hypoxanthin oder aber auch unnatürliche Basen wie beispielsweise Purin, 2.6-Diaminopurin, 7-Deazaguanin, 7-Deazaadenin, N⁴N⁴-Ethanocytosin, N⁶N⁶-Ethano-2.6-diaminopurin, Pseudoisocytosin, 5-Methylcytosin, 5-Fluoruracil, 5-(C₃-C₆)-Alkinyluracil, 5-(C₃-C₆)-Alkinylcytosin oder deren Prodrugformen zu verstehen.

Unter temperaturstabilen Polymeraseenzymen werden Enzyme verstanden, die eine Amplifikation von Nukleinsäuren wie z.B. DNA oder RNA, vorzugsweise von DNA gestatten, ohne daß nach der notwendigen thermischen Strangtrennung nochmals Enzym für weitere Kopiervorgänge zur Verfügung gestellt werden muß.

Im Gegensatz zum Klenow-Enzym, das nur einen einmaligen Kopiervorgang des Template-Stranges zuläßt, weil es bei der anschließenden thermischen Strangtrennung deaktiviert wird, kann mit einer temperaturstabilen Polymerase wie z.B. der Vent™-Polymerase und überschüssigen PNA/DNA-Primer eine Amplifikation des kopierten Templatestranges analog bekannter Amplifikationstechniken wie z. B. PCR (Polymerase Chain Reaction) und LCR (Ligase Chain Reaction) erreicht werden (zu PCR, siehe z. B. Methods in Molecular Biology: 15, (1993) PCR Protocols; Edited by B.A. White, Humana Press).

Die Elongation des PNA/DNA-Primers mit Hilfe thermostabiler DNA-Polymerasen eignet sich besonders gut zur Herstellung multipler Kopien des Templates, wobei das Amplifikations-Produkt bevorzugt radioaktive- oder nichtradioaktive Markergruppen enthält.

Beispielsweise können zur Herstellung radioaktiv markierter Amplifikate [α -³²P]- oder [α-³⁵S]-Deoxynukleosid-5'-triphosphat in die Polymerasereaktion eingesetzt werden. In ähnlicher Weise sind durch Einbau von Nukleosid-triphosphaten, die nichtradioaktive Marker-Gruppen enthalten, entsprechende nichtradioaktiv markierte DNA-Fragmente nach an sich bekannten Methoden (U. Englisch und D. Gauss (1991) Angew. Chem., 103, 629-46.) zugänglich.

Die Amplifikationsreaktion erfolgt beispielsweise nach dem folgenden Schema.

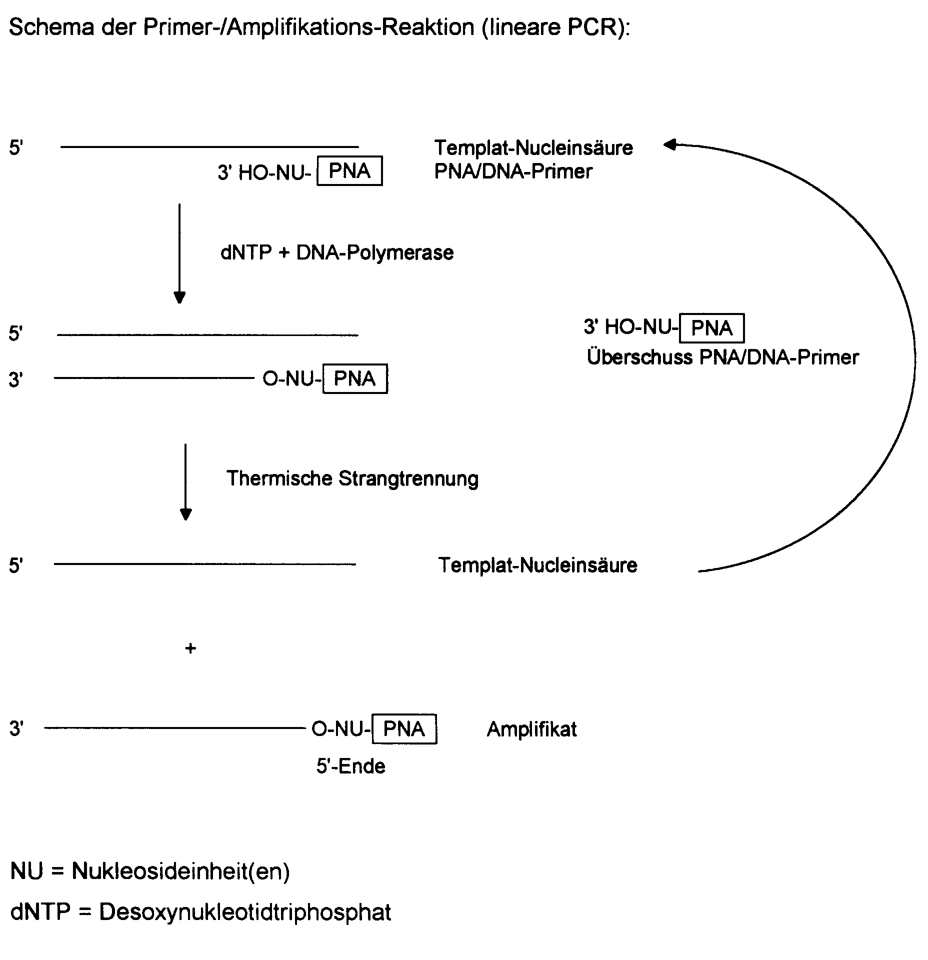
Schema der Primer-/Amplifikations-Reaktion (lineare PCR):

5' Templat-Nucleinsäure
3' HO-NU- PNA PNA/DNA-Primer

dNTP + DNA-Polymerase

5'
3' O-NU- PNA

3' HO-NU- PNA
Überschuss PNA/DNA-Primer

Thermische Strangtrennung

5' Templat-Nucleinsäure

+

3' O-NU- PNA Amplifikat
5'-Ende

NU = Nukleosideinheit(en)
dNTP = Desoxynukleotidtriphosphat

Gemäß dem Fachmann bekannten PCR-Techniken kann mit Hilfe der beschriebenen PNA/DNA-Primer eine exponentielle Amplifikation von geeigneten Template-Nukleinsäuren, insbesondere von Template-DNA durchgeführt werden.

Gegenüber den reinen PNAs, die keine Primerfunktion ausüben können, hat die Verwendung der PNA/DNA-Primer in Kombination mit temperaturstabilen Enzymen wie der Vent ™-Poymerase den weiteren Vorteil, daß die ursprünglich mühsame und wenig sensitive Detektion mittels Kapillarelektrophorese (C. Carlson et al., Nature (1996) 380, 207) durch schnellere und empfindlichere Detektionsmethoden, wie beispielsweise Autoradiographie, PhosphorImager™ oder Antikörper, abgelöst wird.

Ganz generell erstreckt sich die vorliegende Erfindung auch auf die Verwendung in der Diagnostik, z.B. zur Diagnose von Krankheiten, insbesondere in der Diagnose von Krankheiten, die mit der Expression eines oder mehrerer Gene, die mit pathologischen Zuständen verbunden sind, einhergehen.

Mit dem Verfahren kann die Anwesenheit, die Abwesenheit oder die Menge von Target Nucleinsäure Sequenzen nachgewiesen werden.

Die Erfindung betrifft auch die Verwendung des Verfahrens in diagnostischen Tests.

Das Verfahren der vorliegenden Erfindung kann beispielsweise zur Diagnose von Erkrankungen, die durch Viren hervorgerufen werden, beispielsweise durch HIV-, HSV-1, HSV-2, Influenza, VSV, Hepatitis B oder Papilloma-Viren, verwendet werden.

Das Verfahren der vorliegenden Erfindung eignet sich beispielsweise auch zur Diagnose von Krebs. Beispielsweise können dabei Targets diagnostiziert werden, die an der Krebsentstehung bzw. dem Krebswachstum beteiligt sind. Solche Targets sind beispielsweise:
1) Nucleare Onkoproteine wie beispielsweise c-myc, N-myc, c-myb, c-fos, c-fos/jun, PCNA, p120;
2) Cytoplasmische/Membran-assoziierte Onkoproteine wie beispielsweise EJ-ras, c-Ha-ras, N-ras, rrg, bcl-2, cdc-2, c-raf-1, c-mos, c-src, c-abl;
3) Zelluläre Rezeptoren wie beispielsweise EGF-Rezeptor, c-erbA, Retinoid-Rezeptoren, Protein-Kinase regulatorische Untereinheit, c-fms;
4) Cytokine, Wachstumsfaktoren, Extrazelluläre Matrix wie beispielsweise CSF-1, IL-6, IL-1a, IL-1b, IL-2, IL-4, bFGF, Myeloblastin, Fibronectin.

Das Verfahren der vorliegenden Erfindung eignen sich beispielsweise ferner zur Diagnose von Erkrankungen, bei denen Integrine und/oder Zell-Zell-Adhäsionsrezeptoren beteiligt sind, beispielsweise VLA-4, VLA-2, ICAM, VCAM oder ELAM.

Das Verfahren der vorliegenden Erfindung eignen sich beispielsweise auch zur Diagnose von Krankheiten, bei denen die Proliferation und/oder Migration von Zellen eine Rolle spielt. Targets können dann beispielsweise sein:
1) Nucleare Transaktivator-Proteine und Cycline wie beispielsweise c-myc, c-myb, c-fos, c-fos/jun, Cycline und cdc2-Kinase;
2) Mitogene oder Wachstumsfaktoren wie beispielsweise PDGF, bFGF, EGF, VEGF, HB-EGF und TGF-β;
3) Zelluläre Rezeptoren wie beispielsweise bFGF-Rezeptor, EGF-Rezeptor, VEGF-Rezeptor und PDGF-Rezeptor.

Die Vent™ -Polymerase besitzt eine 3'-->5' Exonuclease-Aktivität, welche normale DNA-Primer abbaut (Kong et al., J. Biol. Chem. (1993) 1965-1975). Die erfindungsgemäßen Primer besitzen eine erhöhte Stabilität gegenüber diesem nucleolytischen Abbau.

Versuche mit einer Vielzahl bekannter, kommerziell erhältlicher DNA-Polymerasen, zeigten nun, daß zum Beispiel die temperaturstabilen Tth™ DNA-Polymerase aus Thermus sp., die Pwo DNA-Polymerase aus Pyrococcus woesei, die 9°N DNA-Polymerase™ aus Thermococcus sp. strain 9°N oder die Vent™ -Polymerase aus Thermococcus litoralis in der Lage sind, einen PNA/DNA-Primer, der an einem Ende mindestens eine Nukleosideinheit mit einer 3'-Hydroxygruppe trägt, zu elongieren.

Vorteilhaft ist die Verwendung der Vent ™- Polymerase, da Reaktionen mit Vent ™-Polymerase Kopien von Nukleinsäurefragmenten in voller Länge ergeben.

Vorteilhaft ist des weiteren die Anwendung der Tth™-DNA-Polymerase, da eine hohe Amplifikationsrate erreicht wird.

Für das erfindungsgemäße Verfahren ist es vorteilhaft, wenn der DNA/PNA-Primer gegenüber dem Template im Überschuß vorliegt. Vorteilhaft ist ein 2 bis 20 facher, besonders vorteilhaft ein 5-10 facher Überschuß an DNA/PNA-Primer (jeweils bezogen auf das Molgewicht).

Sequenzen der PNA/DNA-Primer, DNA-Primer und DNA-Template:
PNA/DNA-Primer 1:
   H-taa tac gac tca cta-(5'NH-T)-3'
   (5'NH-T ist 5'-Amino-5'-deoxythymidin, Struktur siehe Formel I, n=0)
PNA/DNA-Primer 2:
   Acetyl-taa tac gac tca cta-(eae(t) A-3'
   (eae(t) ist die Ethylamino-N-(thyminylacetyl)ethanol-Einheit, Struktur siehe Formel III, n=0)
PNA/DNA-Primer 3:
   Acetyl-tgc aag ctt aa (5'NH-T)-3'
   (5'NH-T ist 5'-Amino-5'-deoxythymidin, Struktur siehe Formel I, n=0)
PNA/DNA-Primer 4:
   Acetyl-tgc aag ctt a (PHONA-a) (T)-3'
   (PHONA-a ist die [(2-Aminoethyl)-N9-adenylacetylamino]methyl phosphonsäure-Einheit, Struktur siehe Formel II, n=0)
DNA-Primer 1:
   5'-d(TAA TAC GAC TCA CTA T-3')
DNA-Primer 2:
   5'-d(GCC CCA GGG AGA AGG CAA-3')
DNA-Primer 3:
   5'-d(CGAGCTTAAGTCAGC-3')
DNA-Template 3 (81-mer):
   5'-d(GCC CCA GGG AGA AGG CAA CTG GAC CGA AGG CGC TTG TGG AGA AGG AGT TCA TAG CTG GGC TCC CTA TAG TGA GTC GTA TTA-3')
DNA-Template 4 (46-mer):
   5'-d(CGA GCT TAA GTC AGC GCC TAC TAT AGT GAG TCG TAT TAA GCT TGC A-3')
   - Großbuchstaben:: DNA
   - Kleinbuchstaben:: PNA

### Beispiele

### Beispiel 1:

### Herstellung der Nukleinsäure-Substrate PNA/DNA-Primer 1, DNA-Primer 1 bis 3 und DNA-Template 3 und 4

Der PNA/DNA-Primer 1 (16-mer, H-taatacgactcacta (5'NH-T)- 3') wurde wie in EP 0672 677 A2 beschrieben synthetisiert . Die DNA-Primer 1, 2 und 3 sowie die DNA-Template 3 und 4 wurden an einem ABI Synthesizer synthetisiert und durch Polyacrylamid-Gelelektrophorese gereinigt. Der DNA-Primer 1 (15 pmol) wurde am 5'-Ende unter Verwendung von 10 Einheiten T4-Polynukleotidkinase (Gibco BRL) in einem Gesamtvolumen von 60 µl mit 10 mCi Redivue [γ-³²P]ATP (Amersham, 3000 Ci/mmol) markiert. Die Phosphorylierung erfolgte durch Inkubation bei 37°C für 90 min. Anschließend wurde die Reaktion durch 15 minütiges Erhitzen auf 85°C gestoppt.

### Beispiel 2:

### Elongation von PNA/DNA-Primern mit Hilfe der thermostabilen DNA-Polymerasen Vent™ -Polymerase und Tth™ DNA-Polymerae (Einfüllreaktionen)

Um eine komplette Bindung des Primers an das Template für die Einfüllreaktion sicherzustellen, wurde die Hybridisierung der Primer an das Template in einem Gesamtvolumen von 500 µl, enthaltend 1.8 mM Tris-HCl (pH 7.0), 0.5 mM MgCl₂ und 23 mM NaCl , mit 15 pmol Primer (entweder markierter DNA-Primer 1 oder unmarkierter DNA/PNA-Primer 1) und 50 pmol DNA-Template 3 durchgeführt. Diese Mischung wurde 15 min auf 95°C erhitzt und anschließend für 1h auf Raumtemperatur abgekühlt. Tth™ -DNA Polymerase und Vent™ - Polymerase sind von Boehringer Mannheim und New England Biolabs erhältlich. Die enzymatischen Reaktionen wurden in einem Gesamtvolumen von 25 µl in dem geeigneten Puffer mit 0.15 pmol Primer/Template-Komplex und allen benötigten dNTPs in einer Endkonzentration von a) 5 mM und b) 50 mM durchgeführt. Im Falle des PNA/DNA-Primers 1 wurde unmarkiertes dCTP durch [α -³²P]-dCTP (400 Ci/mmol) ersetzt. Falls MgCl₂ oder MgSO₄ für die enzymatische Reaktion notwendig waren, wurde die Verbindung in einer Endkonzentration von 2 mM zum Reaktionsgemisch gegeben. Durch Zugabe des Enzyms wurde die Reaktion gestartet und 15 min entweder bei 37°C oder bei 75°C inkubiert. Anschließend wurde durch Zugabe von 5 µl eines stop/loading-Farbstoffs (New England Biolabs) die Reaktion unterbrochen. Aliquote Teile jeder Reaktion (5 µl) wurden mittels Polyacrylamid-Gelelektrophorese an nativem Gel (Gelstärke 12%, 10 Watt für 4 h) und denaturierendem Gel (Gelstärke 15%, 35 Watt für 2 h) analysiert. Die Gele wurden anschließend in einer wäßrigen Lösung von 12 % (v/v) Methanol und 10 % (v/v) Essigsäure fixiert, getrocknet und mittels Autoradiographie durch über Nacht Belichtung einer PhosphorImager™ -Platte (Molecular Dynamics) untersucht.

### Beispiel 3:

### Lineare PCR-Reaktionen (Amplifikation)

Zur Amplifikation des Templatestranges mit Hilfe der Vent ™ DNA-Polymerase und des PNA/DNA-Primers 1 wurde ein Überschuß an Primer von 20 pmol PNA/DNA-Primer 1 mit 2 pmol DNA-Template 3 (81-mer) und 200 mM dNTPs in einem Endvolumen von 50 µl geeignetem Reaktionspuffer gemischt. Die Mischung wurde mit Mineralöl überschichtet und die Reaktion durch Zugabe von 2 Einheiten Vent ™ DNA-Polymerase gestartet. Die Polymerase Reaktion wurde folgendermaßen durchgeführt: 30 Zyklen 1 min 94°C, 2 min 58°C, 50 sec 72°C und zuletzt zur Vervollständigung der Reaktion 8 min bei 72°C. Die Mischung wurde einmal mit Phenol, dann mit Phenol/Chloroform und zum Schluß mit Chloroform extrahiert. Die DNA wurde mit 7.8 M Ammoniumacetat und 100 % (v/v) Ethanol ausgefällt. Der Niederschlag wurde in 10 µl TE-Puffer gelöst und ein Aliquot mit Hilfe der Gelelektrophorese analysiert.

### Beispiel 4:

### N-(2-Monomethoxytritylamino)ethylaminoethanol

Zu einer gerührten Lösung von 9,3 g N-(2-Hydroxyethyl)diaminoethan in 100 ml wasserfreiem Dimethylformamid bei 0°C wird eine Lösung von 9,24 g Monomethyoxytritylchlorid in 60 ml Dichlormethan innerhalb von 2 Stunden zugegeben. Das Gemisch wird bei 4°C über nacht gerührt. Anschließend wird das Lösungsmittel unter Vakuum entfernt, und der Rückstand wird zwischen 100 ml Ethylacetat und 50 ml Wasser verteilt. Die wäßrige Phase wird viermal mit 40 ml Ethylacetat eingedampft. Das zurückbleibende rohe Produkt wird mittels Chromatographie auf Kieselgel mit einem Gradienten von (1-10%) Methanol in Ethylacetat, der 1% Triethylamin enthält, gereinigt. Die das reine Produkt enthaltenden Fraktionen werden vereint und zur Trockne eingedampft.
Ausbeute: 7,91 g Schaum.
R_{f}: 0,14 (Ethylacetat/Methanol/Triethylamin: 100/10/1)
MS(FAB, MeOH/NBA/LiCl): 383,3 (M+Li)⁺

### Beispiel 5:

### N-2-(Monomethoxytritylamino)ethylamino-N-(thyminylacetyl)ethanol

1,84 g Carboxymethylthymin werden in 50 ml wasserfreiem Dimethylformamid gelöst, dann werden 3,24 g TOTU und 3,4 ml Diisopropylethylamin zugegeben. Das Gemisch wird 20 Minuten lang gerührt, und anschließend werden 3,76 g N-(2-Monomethoxytritylamino)ethylaminoethanol aus Beispiel 4 zugegeben. Die Lösung wird 16 Stunden lang bei Raumtemperatur gerührt und anschießend unter Vakuum eingedampft. Der Rückstand wird in 80 ml Ethylacetat gelöst und anschließend viermal mit 20 ml Wasser extrahiert, in dem 0,5% Triethylamin enthalten sind. Die organische Phase wird über Natriumsulfat getrocknet und auf etwa 15 ml eingeengt. Das Produkt wird durch Zutropfen in 200 ml Diethylether unter Rühren ausgefällt. Das rohe Produkt wird abfiltriert und getrocknet. Das Produkt wird mittels Chromatographie auf Kieselgel mit einem Gradienten von (2-5%) Methanol in Ethylacetat/Heptan (10/1), worin 1% Triethylamin enthalten ist, weiter gereinigt. Die das reine Produkt enthaltenden Fraktionen werden vereint, zur Trockne eingedampft und mit Diethylether trituriert.
Ausbeute: 1,95 g amorpher Feststoff.
R_{f}: 0,46 (Ethylacetat/Methanol/Triethylamin: 100/20/1)
MS(ES⁺): 543,3 (M+H)⁺

### Beispiel 6:

### Diisopropylphosphoramidonsäure-2-cyanoethylester-2-(2-[(4-methoxyphenyl)diphenylmethyl]amino ethyl)-[(5-methyl-2,4-dioxo-3,4-dihydro-2H-pyrimidin-1-yl)acetyl]aminoethylester

N-(2-Hydroxyethyl)-N-(2-[(4-methoxyphenyl)diphenylmethyl]aminoethyl)-2-(5-methyl-2,4-dioxo-3,4-dihydro-2H-pyrimidin-1-yl)acetamid aus Beispiel 5 (0,5 g; 0,922 mMol) wird in wasserfreiem Dichlormethan (15 ml) gelöst. Zu dieser Lösung werden N,N-Diisopropylethylamin (3,69 mMol; 0,63 ml) und 2-Cyanoethyl-N,N-diisopropylphosphoramidochloridit (1,10 mMol; 0,24 ml) zugegeben. Nach einer Stunde wird Ethylacetat zu dem Reaktionsgemisch zugegeben, und das Lösemittel wird unter Vakuum verdampft. Der Rückstand wird in Ethylacetat gelöst und viermal mit gesättigter wäßriger NaCl-Lösung gewaschen. Die organische Phase wird getrocknet (Na₂SO₄), filtriert und unter Vakuum eingedampft. Der Rückstand wird mittels Säulenchromatographie auf Kieselgel mit Dichlormethan:Ethylacetat:NEt₃ (49,5:49,5:1) als Elutionsmittel gereinigt. Die produkthaltigen Fraktionen werden vereint und eingeengt; man erhält die in der Überschrift aufgeführte Verbindung als weißen Feststoff in einer Ausbeute von 0,474 g (69%).
R_{f} = 0,45 (Dichlormethan:Ethylacetat / 1:1); ³¹P-NMR (CDCl₃) d 133,75 (s), 134,5 (s).
MS (FAB): 743,4 (M⁺)

### Beispiel 7:

### PNA/DNA-Primer 2, Acetyl-taa tac gac tca cta-(eae(t) A-3'

Eine mit handelsüblichem Adenosin-derivatisiertem Glas mit kontrollierter Porengröße (5 mMol) gefüllte Synthesesäule wird auf einen Eppendorf Biotronik Ecosyn D 300 DNA-Synthesizer aufgesetzt, und die 5'-terminale Dimethoxytrityl-Gruppe wird durch Behandlung mit einer 3%igen Trichloressigsäure-Lösung in Dichlormethan entfernt. Nach gründlichem Waschen mit Acetonitril wird eine mit einer DNA-Aktivatorlösung (Tetrazol in Acetonitril, Applied Biosystems) gemischte 0,1 M Lösung von Ethylamino-N-(thyminylacetyl)ethanolphosphoramidit auf die Säule gegeben. Man läßt die Kopplungsreaktion 15 Minuten lang laufen, dann wird sie wiederholt. Die Säule wird mit Acetonitril gewaschen, dann wird DNA-Oxidationslösung (Jod, Pyridin, Wasser; Applied Biosystems) für eine Minute zu der Säule zugegeben. Nach dem Waschen werden die nicht umgesetzten Hydroxylfunktionen durch Capping mit Essigsäureanhydrid, N-Methylamidazol, Lutidin in THF (Applied Biosystems) geschützt. Nach gründlichem Waschen mit Acetonitril wird die N-Monomethoxytrityl-Schutzgruppe durch Behandlung mit einer 3%igen Trichloressigsäure-Lösung in Dichlormethan entfernt. Die PNA-Synthese wird dann wie in EP 0672 677 A2 beschrieben fortgesetzt. Nach Abschluß der Synthese wird die N-terminale Monomethoxytrityl-Schutzgruppe durch Behandlung mit einer 3%igen Trichloressigsäure-Lösung in Dichlormethan entfernt, und der Amino-Terminus wird durch Capping mit Essigsäureanhydrid, N-Methylimidazol, Lutidin in THF (Applied Biosystems) geschützt. Der Primer wird mit Hilfe von konzentrierter wäßriger Ammoniaklösung von dem festen Substrat abgespalten, und die basischen Schutzgruppen werden durch Erhitzen dieser Lösung auf 55°C während 5 Stunden entfernt. Ein Teil der Probe wird mittels semipräparativer C₁₈ Umkehr-Phasen-HPLC mit 10-80% MeCN in 0,1 M Triethylammoniumacetat als Elutionsmittel gereinigt. Nach Entsalzung auf einer Sephadex NAP-10 Säule (Pharmacia) und Lyophilisierung erhält man den PNA/DNA-Primer 2 in einer Ausbeute von 2 OD₂₆₀ (= 10,38 nMol). MS (MALDI-TOF) m/z 4636,1 (berechnet 4635,41).

### Beispiel 8:

### PNA/DNA-Primer 3, Acetyl-tgc aag ctt aa (5'NH-T)-OH 3'

PNA/DNA-Primer 3 (12-mer, Acetyl-tgc aag ctt aa (5'NH-T)-OH 3') wird analog zu PNA/DNA-Primer 1 wie in EP 0672 677 A2 beschrieben synthetisiert. Nach der Entfernung der Monomethoxytrityl-Gruppe der letzten PNA-Einheit wird jedoch wie in Beispiel 7 beschrieben ein Capping vorgenommen.

### Beispiel 9:

### PNA/DNA-Primer 4, Acetyl-tgc aag ctt a (PHONA-a) (T)-OH-3'

PNA/DNA-Primer 4 wird ausgehend von dem mit Thymidin beladenen CPG-Substrat synthetisiert. Die Dimethoxytrityl-Gruppe wird durch Behandlung mit einer 3%igen Trichloressigsäurelösung in Dichlormethan entfernt. Der PHONA-a Monomerbaustein wird wie in EP 0739898 beschrieben hergestellt und an die Hydroxylgruppe gekoppelt. Die PNA-Synthese wird dann wie in EP 0672 677 A2 beschrieben fortgesetzt.

### Beispiel 10:

### Auffüll-Reaktion unter Verwendung von PNA/DNA-Primer 1, 81-mer DNA-Template 3 und Pwo-DNA-Polymerase (Pyrococcus woesei)

Der PNA/DNA-Primer 1 (15 pMol) wurde in einem Gesamtvolumen von 500 µl, in dem 1,8 mM Tris-HCl (pH 7,0), 0,5 mM MgCl₂ und 23 mM NaCl enthalten waren, mit dem DNA-Template 3 (50 pMol) hybridisiert. Die Auffüll-Reaktionen wurden in dem für Pwo-DNA-Polymerase bereitgestellten Puffer (25 µl) durchgeführt, der 0,15 pMol Primer/Template-Komplex und alle erforderlichen dNTPs in einer Endkonzentration von 50 µMol enthielt. Nicht markierte dCTP wurde durch [α-³²P]-dCTP (400 Ci/mMol) ersetzt.

Die Reaktion wurde durch Zugabe von 2 Einheiten Pwo-DNA-Polymerase (Pyrococcus woesei) (Lieferant: Boehringer Mannheim, Deutschland) eingeleitet und 15 Minuten lang bei 75°C inkubiert. Aliquote Teile von jeder Reaktion (5 µl) wurden sowohl durch native (12% Acrylamid, 10 Watt, 4 Stunden) als auch nach 20-minütigem Erhitzen auf 95°C durch denaturierende (15% Acrylamid, 35 Watt, 2 Stunden) PAGE (Polyacrylamid-Gelelektrophorese) analysiert. Die Gele wurden fixiert, getrocknet und einer Autoradiographie unterzogen (Phosphorlmager, Molecular Dynamics).

### Beispiel 11:

### Auffüllreaktion unter Verwendung von PNA/DNA-Primer 1, 81-mer DNA-Template 3 und 9°N DNA-Polymerase™.

Die Auffüllreaktion wird wie in Beispiel 10 beschrieben durchgeführt, jedoch wird 9°N DNA-Polymerase™ (New England Biolabs ) in dem entsprechenden Puffer eingesetzt.

### Beispiel 12:

### Auffüllreaktion unter Verwendung von PNA/DNA-Primer 2, 81-mer DNA-Template 3 und Pwo-DNA-Polymerase (Pyrococcus woesei).

Die Auffüllreaktion wird analog zu Beispiel 10 durchgeführt, jedoch wird PNA/DNA-Primer 2 eingesetzt.

### Beispiel 13:

### Auffüllreaktion unter Verwendung von PNA/DNA-Primer 2, 81-mer DNA-Template 3 und 9°N DNA-Polymerase™.

Die Auffüllreaktion wird analog zu Beispiel 11 durchgeführt, jedoch wird PNA/DNA-Primer 2 eingesetzt.

### Beispiel 14:

### Polymerase-Kettenreaktion (PCR) unter Verwendung von PNA/DNA-Primer 3, DNA-Primer 3, 46-mer DNA-Template 4 und Tth™-DNA-Polymerase.

Für die PCR werden 50 pMol PNA/DNA-Primer 3 von Beispiel 8, 5 pMol DNA-Template 4, 0,5 pMol 5'-markierter DNA-Primer 3 und 200 µM dNTPs in einem Endvolumen von 50 µl gemischt, in dem der Tth-DNA-Polymerase-Reaktionspuffer und 2 Einheiten Tth-DNA-Polymerase enthalten sind. Die anderen Bedingungen sind wie in Beispiel 10 beschrieben. Die Amplifikation wird wie folgt durchgeführt: 30 Zyklen, 1 Minute 94°C, 2 Minuten 58°C, 50 Sekunden 72°C.

### Beispiel 15:

### Polymerase-Kettenreaktion (PCR) unter Verwendung von PNA/DNA-Primer 4, DNA-Primer 3, 46-mer DNA-Template 4 und Tth™-DNA-Polymerase

Für die PCR werden 50 pMol PNA/DNA-Primer 4 von Beispiel 9, 5 pMol DNA-Template 4, 0,5 pMol 5'-markierter DNA-Primer 3 und 200 µM dNTPs in einem Endvolumen von 50 µl gemischt, in dem der Tth-DNA-Polymerase-Reaktionspuffer und 2 Einheiten Tth™-DNA-Polymerase enthalten sind. Die anderen Bedingungen sind wie in Beispiel 10 beschrieben. Die Amplifikation wird wie folgt durchgeführt: 30 Zyklen, 1 Minute 94°C, 2 Minuten 58°C, 50 Sekunden 72°C.

### Beispiel 16:

### Polymerase-Kettenreaktion (PCR) unter Verwendung von PNA/DNA-Primer 1, 81-mer DNA-Template 3 und Tth™-DNA-Polymerase

Für die PCR werden 50 pMol PNA/DNA-Primer 1 von Beispiel 1, 5 pMol DNA-Template 3 (siehe Beispiel 1), 0,5 pMol 5'-markierter DNA-Primer 2 und 200 µM dNTPs in einem Endvolumen von 50 µl gemischt, in dem der Tth-DNA-Polymerase-Reaktionspuffer und 2 Einheiten Tth™-DNA-Polymerase enthalten sind. Die anderen Bedingungen sind wie in Beispiel 10 beschrieben. Die Amplifikation wird wie folgt durchgeführt: 30 Zyklen, 1 Minute 94°C, 2 Minuten 58°C, 50 Sekunden 72°C.

### Beispiel 17:

### Polymerase-Kettenreaktion (PCR) unter Verwendung von PNA/DNA-Primer 1, 81-mer DNA-Matrize 3 und 9°N-DNA-Polymerase™.

Die PCR wird wie in Beispiel 16 beschrieben durchgeführt, jedoch wird 9°N-DNA-Polymerase™ eingesetzt.

## Patentansprüche

1. Verfahren zur Amplifikation von Nukleinsäuren mit Hilfe eines oder mehrerer PNA/DNA-Primer, die an einem Ende mindestens eine Nukleosid-Einheit mit einer 3 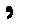 -Hydroxygruppe haben, eines Polymeraseenzyms sowie den üblicherweise benötigten Komponenten, dadurch gekennzeichnet, daß das Polymeraseenzym temperaturstabil ist.

2. Verfahren nach Anspruch 1, dadurch gekennzeichnet, daß das Polymeraseenzym ein Enzym aus der Reihe Vent ™-Polymerase, Pwo-DNA-Polymerase, Tth™-DNA-Polymerase oder 9°N DNA-Polymerase™ ist.

3. Verfahren nach einem oder mehreren der Ansprüche 1 und 2, dadurch gekennzeichnet, daß eine Kombination mehrerer Polymeraseenzyme verwendet wird.

4. Verfahren nach Anspruch 3, dadurch gekennzeichnet, daß eine Kombination der Polymeraseenzyme Vent™ und Tth™ DNA Polymerase verwendet wird.

5. Verfahren nach einem oder mehreren der Ansprüche 1 bis 4, dadurch gekennzeichnet, daß der PNA/DNA-Primer an einem Ende ein bis drei Nukleosid-Einheiten aufweist.

6. Verfahren nach einem oder mehreren der Ansprüche 1-5, dadurch gekennzeichnet, daß der PNA/DNA-Primer an einem Ende eine Nukleosid-Einheit aufweist.

7. Verfahren nach einem oder mehreren der Ansprüche 1-6, dadurch gekennzeichnet, daß DNA amplifiziert wird.

8. Verfahren nach einem oder mehreren der Ansprüche 1-7, dadurch gekennzeichnet, daß sich die Nukleosideinheiten am 3'-Ende des PNA/DNA-Primers befinden.

9. Verwendung eines temperaturstabilen Polymeraseenzyms in einem Verfahren zur Amplifikation von Nukleinsäuren gemäß einem oder mehreren der Ansprüche 1-8.

10. Verwendung gemäß Anspruch 9, dadurch gekennzeichnet, daß als temperaturstabiles Enzym ein Enzym aus der Reihe Vent ™-Polymerase, Pwo-DNA-Polymerase, Tth™-DNA-Polymerase oder 9°N DNA-Polymerase™ eingesetzt wird.

11. Verwendung des Verfahrens gemäß einem oder mehreren der Ansprüche 1 bis 8 in einem Diagnostikum.


SEQUENZPROTOKOLL

(1) ALLGEMEINE INFORMATION:

(i) ANMELDER:
(A) NAME: Hoechst Marion Roussel Deutschland GmbH
(B) STRASSE: -
(C) ORT: Frankfurt
(D) BUNDESLAND: -
(E) LAND: Deutschland
(F) POSTLEITZAHL: 65926
(G) TELEPHON: 069-305-7072
(H) TELEFAX: 069-35-7175
(I) TELEX: -

(ii) ANMELDETITEL: Verfahren zur Amplifikation von
Nukleinsaeuren

(iii) ANZAHL DER SEQUENZEN: 9

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 16 Basenpaare
(B) ART: Polyamid-Nukleinsäuren
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..16
(D) SONSTIGE ANGABEN: /note= "N 16 = (5'NH-T)"

(xi) SEQUENZBESCHREIBUNG: SEQ ID NO: 1:

TAATACGACT CACTAN 16

(2) INFORMATION ZU SEQ ID NO: 2:

(i) SEQUENZ CHARAKTERISTIKA:
(A) LÄNGE: 18 Basenpaare
(B) ART: Polyamid-Nukleinsäuren, Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..18
(D) SONSTIGE ANGABEN: /note= "N1 = Acetyl-; N17 =

(eae(t)"

(xi) SEQUENZBESCHREIBUNG: SEQ ID NO: 2:

NTAATACGAC TCACTANA                                                    18

(2) INFORMATION ZU SEQ ID NO: 3:

(i) SEQUENZ CHARAKTERISTIKA:
(A) LÄNGE: 13 Basenpaare
(B) ART: Polyamid-Nukleinsäuren
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..13
(D) SONSTIGE ANGABEN: /note= "N1 = Acetyl-; N13 =
(5'NH-T)"

(xi) SEQUENZBESCHREIBUNG: SEQ ID NO: 3:

NTGCAAGCTT AAN                                                         13

(2) INFORMATION ZU SEQ ID NO: 4:

(i) SEQUENZ CHARAKTERISTIKA:
(A) LÄNGE: 13 Basenpaare
(B) ART: Polyamid-Nukleinsäuren, Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..13
(D) SONSTIGE ANGABEN: /note= "N1 = Acetyl-; N12 =
(PHONA-a)"

(xi) SEQUENZBESCHREIBUNG: SEQ ID NO: 4:

NTGCAAGCTT ANT                                                         13

(2) INFORMATION ZU SEQ ID NO: 5:

(i) SEQUENZ CHARAKTERISTIKA:
(A) LÄNGE: 16 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..16

(xi) SEQUENZBESCHREIBUNG: SEQ ID NO: 5:

TAATACGACT CACTAT 16

(2) INFORMATION ZU SEQ ID NO: 6:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..18

(xi) SEQUENZBESCHREIBUNG: SEQ ID NO: 6:

GCCCCAGGGA GAAGGCAA 18

(2) INFORMATION ZU SEQ ID NO: 7:

(i) SEQUENZ CHARAKTERISTIKA:
(A) LÄNGE: 15 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..15

(xi) SEQUENZBESCHREIBUNG: SEQ ID NO: 7:

CGAGCTTAAG TCAGC 15

(2) INFORMATION ZU SEQ ID NO: 8:

(i) SEQUENZ CHARAKTERISTIKA:
(A) LÄNGE: 81 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:

(A) NAME/SCHLÜSSEL: exon
(B) LAGE: 1..81

(xi) SEQUENZBESCHREIBUNG: SEQ ID NO: 8:

GCCCCAGGGA GAAGGCAACT GGACCGAAGG CGCTTGTGGA GAAGGAGTTC ATAGCTGGGC 60

TCCCTATAGT GAGTCGTATT A 81

(2) INFORMATION ZU SEQ ID NO: 9:

(i) SEQUENZ CHARAKTERISTIKA:
(A) LÄNGE: 46 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..46

(xi) SEQUENZBESCHREIBUNG: SEQ ID NO: 9:

CGAGCTTAAG TCAGCGCCTA CTATAGTGAG TCGTATTAAG CTTGCA 46